(19) Europäisches Patentamt
European Patent Office
Office européen des brevets

(11) EP 3 006 086 A1

(12) EUROPEAN PATENT APPLICATION
published in accordance with Art. 153(4) EPC

(43) Date of publication:
13.04.2016 Bulletin 2016/15

(51) Int Cl.:
A61N 7/02 (2006.01)          A61B 8/00 (2006.01)

(21) Application number: 13885703.2

(22) Date of filing: 31.05.2013

(86) International application number:
PCT/KR2013/004835

(87) International publication number:
WO 2014/193012 (04.12.2014 Gazette 2014/49)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME

(71) Applicant: Alpinion Medical Systems Co., Ltd.
Guro-gu, Seoul, 152-848 (KR)

(72) Inventors:
• SON, Keonho
Bundang-gu, Seongnam-si
Gyeonggi-do 463-440 (KR)

• KANG, Kookjin
Suji-gu, Yongin-si
Gyeonggi-do 448-536 (KR)
• KIM, Daeseung
Seoul 157-882 (KR)
• KIM, Myungdeok
Seoul 152-020 (KR)

(74) Representative: Thorniley, Peter et al
Kilburn & Strode LLP
20 Red Lion Street
London WC1R 4PJ (GB)

(54) TRANSDUCER STRUCTURE FOR IMPROVING IMAGE QUALITY

(57) Some embodiments of the present disclosure provide a transducer including a high intensity focused ultrasound (HIFU) transducer including a HIFU emitting unit configured to emit a treatment ultrasound and an imaging transducer arranged in proximity of the HIFU transducer and configured to emit an imaging ultrasound. With a parabolic shape, the HIFU emitting unit has an inner circumferential surface formed with a plurality of recesses for extinguishing the treatment ultrasound or the imaging ultrasound, back-reflected to the HIFU emitting unit.

FIG. 2

EP 3 006 086 A1

**Description**

[Technical Field]

The present disclosure relates to a transducer, and more particularly, to a transducer for improving image quality of an image acquired by an imaging transducer.

[Background]

The statements in this section merely provide background information related to some embodiments of the present disclosure and do not necessarily constitute prior art.

An ultrasound transducer is configured to emit an ultrasound to a treatment site and to receive an echo ultrasound which is a reflected ultrasound from a subject to be treated, in order to obtain an ultrasound image of the subject or to treat the subject.

The ultrasound transducer can be applied to various industrial fields. In particular, the ultrasound transducer is mainly employed in a field of a medical apparatus such as an ultrasound medical apparatus for emitting an ultrasound to a treatment site inside a subject from an outer surface of the human body to treat the treatment site with the emitted ultrasound or to obtain a tomographic image of a soft tissue or an image of blood flow by using a reflected ultrasound in a noninvasive manner.

The ultrasound transducer uses the characteristics of a piezoelectric member to generate the ultrasound. The piezoelectric member is a material that converts an electrical energy into a mechanical energy, and vice versa. For example, the piezoelectric member used in the ultrasound transducer includes electrodes formed respectively on an upper portion and a lower portion thereof, and when a power is applied to the electrodes, the piezoelectric member oscillates to interconvert the electrical signal and the mechanical signal.

In general, the ultrasound transducer includes an imaging transducer for acquiring an image of a subject, a HIFU (High Intensity Focused Ultrasound) transducer for treating the subject, and an integrated ultrasound transducer including both the imaging transducer and the HIFU transducer for simultaneously performing a diagnosis and a treatment.

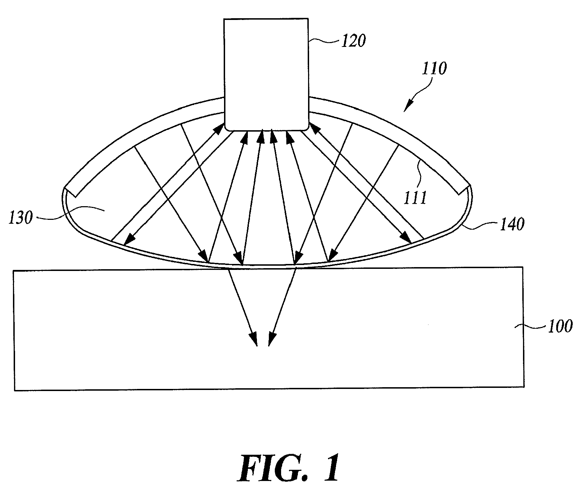

FIG. 1 is a schematic diagram of an integrated transducer including an imaging transducer 120 and a HIFU transducer 110.

Such an integrated transducer includes the HIFU transducer 110 for emitting a treatment ultrasound, the imaging transducer 120 which is located centrally of the HIFU transducer 110 and emits an image acquisition ultrasound or imaging ultrasound. The HIFU transducer 110 and the imaging transducer 120 have a space on their front side filled with a fluid medium 130, such as water, in order to help the ultrasound emitted forward to arrive at a subject 100, and have a membrane 140 for preventing a leakage of the fluid medium 130.

The HIFU transducer 110 includes a HIFU emitting unit 111 for emitting the ultrasound. It is ideal that the treatment ultrasound emitted from the HIFU emitting unit 111 not return to the HIFU emitting unit 111 by reflections at the subject 100, the membrane 140, and the like. Further, it is ideal that the imaging ultrasound emitted from the imaging transducer 120 return to the imaging transducer 120 by reflection at the treatment site of the subject 100 but not return to the imaging transducer 120 by reflections at the membrane 140 and the like.

However, once emitted, the treatment ultrasound or the imaging ultrasound is reflected at a boundary of the fluid medium 130, the membrane 140, a surface of the subject 100, or the like, a portion of the reflected ultrasound repeats the reflection to be re-incident to the imaging transducer 120, and such a re-incident ultrasound undesirably degrades the ultrasound image.

The ultrasound transducer has its unique impedance while the subject 100 has its own impedance, and hence an impedance matching is required to eliminate the impedance difference, for which the ultrasound transducer includes matching layers which will be described later.

However, in the integrated ultrasound transducer, if the impedance matching layers of the HIFU transducer 110 and the imaging transducer 120 are of mismatched thicknesses, some imaging ultrasonic waves, reflected at the membrane 140 and directed toward the HIFU transducer 110, are unable to penetrate the matching layer of the HIFU transducer 110 but further reflected at a surface thereof before they are eventually incident on the imaging transducer 120. Such imaging ultrasound that is re-incident on the imaging transducer 120 without penetrating the subject 100 undesirably degrades the quality of the ultrasound image.

[Disclosure]

[Technical Problem]

The present disclosure has been made in view of the above aspects, and it is an object of the present disclosure to provide a structure for improving the quality of the ultrasound image acquired by the imaging transducer in an integrated ultrasound transducer including a HIFU transducer and an imaging transducer.

The technical problem to be solved by the present disclosure is not limited to the above-mentioned, and other technical problems not mentioned herein can be clearly understood by one of ordinary skill in the pertinent art from the following descriptions.

[Summary]

A transducer according to some embodiments of the present disclosure includes a high intensity focused ultrasound (HIFU) transducer including a HIFU emitting unit configured to emit a treatment ultrasound and an imaging transducer arranged in proximity of the HIFU transducer and configured to emit an imaging ultrasound. The HIFU emitting unit may include at least one recess configured to extinguish the treatment ultrasound and the imaging ultrasound, back-reflected to the HIFU emitting unit. Each of the recesses may includes aformation of a cylindrical shape with an open axial side.

Each of all or some of the recesses may include a first piezoelectric member configured to generate the treatment ultrasound, and the imaging transducer includes a second piezoelectric member configured to generate the imaging ultrasound. The at least one recess includes a filling member on an outer area of the recess, the first piezoelectric member on an inner side of the recess, and a first matching layer, between the first piezoelectric member and the filling member, for matching an acoustic impedance of the first piezoelectric member with an acoustic impedance of a subject at which the ultrasound arrives. The imaging transducer may include a second matching layer for matching an acoustic impedance of the second piezoelectric member with the acoustic impedance of the subject. The filling member may include rubber or polymer material.

[Advantageous Effects]

With the above-described transducer according to some embodiments of the present disclosure, the quality of the ultrasound image can be improved by extinguishing the ultrasound back-reflected to the HIFU emitting unit or adjusting the thickness of the matching layer.

[Brief Description of Drawings]

FIG. 1 is a schematic diagram of an integrated transducer including an imaging transducer and a HIFU transducer.
FIG. 2 is a cross-sectional view of a transducer according to some embodiments of the present disclosure.
FIG. 3 is a front view of a HIFU emitting unit of a transducer according to some embodiments of the present disclosure.
FIG. 4 is a partial cross-sectional view of a HIFU transducer of a structure according to some embodiments of the present disclosure.

REFERENCE NUMERALS

| 100: | subject | 210: | HIFU transducer |
|---|---|---|---|
| 211: | HIFU emitting unit | 2110: | recess |
| 2111: | first piezoelectric member | 2112: | first matching layer |
| 2112-1: | surface | 2113: | filling member |
| 220: | imaging transducer | 221: | second piezoelectric member |
| 222: | second matching layer | 230: | fluid medium |
| 240: | membrane | 310: | hollow portion |
| 400: | back-reflected ultrasound | | |

[Detailed Description]

Hereinafter, at least one embodiment of the present disclosure will be described in detail with reference to the accompanying drawings. The present disclosure contemplates various changes and modifications to be made, although they are illustrated through some exemplary embodiments. The present disclosure should not be limited to these embodiments but various changes and modifications are made by one ordinarily skilled in the art within the subject matter, the idea and scope of the present disclosure as hereinafter claimed. In the following description, like reference numerals designate like elements, although the elements are shown in different drawings. In the accompanying drawings, structures are exaggerated to emphasize some embodiments of the disclosure or reduced to facilitate the comprehension thereof.

Terms such as first and second, which may be used to describe various components, should not be interpreted as limiting said components. The above terms are used only to distinguish one of the components from the others. For example, and without departing from the scope of the present disclosure, the first component can be designated as the second component, and vice versa. On the other hand, unless defined otherwise, all terms, including technical or scientific terms used herein have the same meaning as are generally understood by persons of skill in the art to which this disclosure pertains. The terms, such as those commonly used as in lexical definition, should be interpreted as having a meaning consistent with the meaning that has the context of the relevant art, and unless expressly defined in this application, they shall not be interpreted too ideally or impractically unless the present disclosure expressly defines them so.

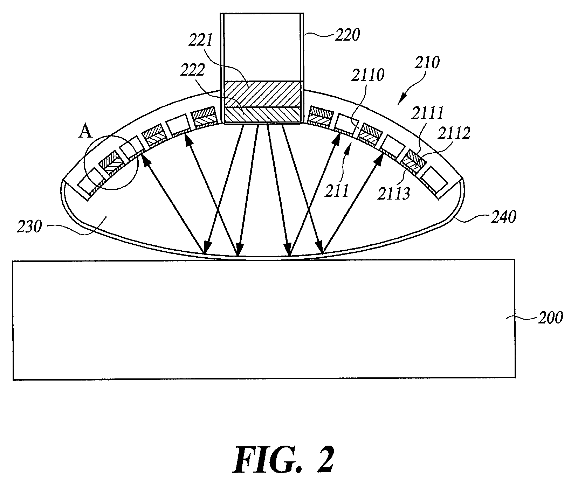

FIG. 2 is a cross-sectional view of a transducer according to some embodiments of the present disclosure.

A HIFU transducer 210 emits a treatment ultrasound to a subject 200. The HIFU transducer 210 includes a HIFU emitting unit 211. In some embodiments, the HIFU emitting unit 211 is formed in a parabolic shape, emits the treatment ultrasound forward from an inner circumferential surface of the parabolic shape, and includes a plurality of recesses 2110 on the inner circumferential surface.

Each of the recesses 2110 is formed, for example, in a circular or polygonal shape with an open axial side, and is provided with a first piezoelectric member 2111, a first matching layer 2112 and a filling member 2113. The first piezoelectric member 2111 is disposed on the inner side of each of the recesses 2110, includes a piezoelectric material, and generates the treatment ultrasound with a power supplied from an external power source. The first matching layer 2112 is interposed between the first piezoelectric member 2111 and the filling member 2113, and prevents the ultrasound from being reflected at the subject 200 by matching an acoustic impedance of the first piezoelectric member 2111 with that of the subject 200. The filling member 2113 prevents a bubble generated by a fluid medium 230 when exposed to the ultrasound from penetrating into the recess 2110. In some embodiments, the filling member 2113 includes a light and corrosion-resistant material against the fluid medium 230 such as water, e.g., rubber or polymer material. FIG. 2 shows that the filling member 2113 is individually inlaid to a outer area of each of the recesses 2110, although this scheme is merely illustrative. In some embodiments, the filling member 2113 is formed in a parabolic shape corresponding to the inner circumferential surface of the HIFU emitting unit 211 and applied to the entire inner circumferential surface of the HIFU emitting unit 211, or formed in a structure that serves to prevent the bubble from entering the recesses 2110 and is mounted on the inner circumferential surface of the HIFU emitting unit 211.

The recesses 2110 extinguish any back-reflected ultrasound that is some of the ultrasound generated from the first piezoelectric member 2111 or a second piezoelectric member 221, emitted to the subject 200, and then back-reflected to the HIFU emitting unit 211 after being reflected at boundaries of the fluid medium 230, a membrane 240, a surface of the subject 200, and the like. That is, when the back-reflected ultrasound is incident on the recesses 2110 and collides with their side walls and bottom walls, the back-reflected ultrasound is extinguished and an energy of the back-reflected ultrasound is converted into a frictional heat. This operation of the recesses 2110 for effectively extinguishing the back-reflected ultrasound returned to the HIFU emitting unit 211 can prevent the back-reflected ultrasound from reentering an imaging transducer 220 and in turn degrading the quality of the ultrasound image.

The first piezoelectric member 2111 and the first matching layer 2112 are not necessarily arranged in all of the recesses 2110. When the first piezoelectric member 2111 and the first matching layer 2112 are arranged in the recesses 2110, the back-reflected ultrasound may be re-reflected at boundaries of the first piezoelectric member 2111 and the first matching layer 2112, inhibiting the performance of the recesses 2110 to extinguish the back-reflected ultrasound. Therefore, in some embodiments, appropriate numbers of the first piezoelectric member 2111 and the first matching layer 2112 are arranged in the recesses 2110 in select positions. In this case, the recesses 2110 free of the first piezoelectric member 2111 and the first matching layer 2112 are dedicated to extinguishing the back-reflected ultrasound. On the other hand, the recesses 2110 including the first piezoelectric member 2111 and the first matching layer 2112 emit the ultrasound, and at the same time, extinguish the back-reflected ultrasound.

However, the filling member 2113 is disposed in all of the recesses 2110 for the purpose of preventing the bubble generated in the fluid medium 230 from entering the recesses 2110.

Configured to emit an ultrasound for acquiring an image of the subject 200, the imaging transducer 220 includes the second piezoelectric member 221 and a second matching layer 222. The second piezoelectric member 221 is disposed in the imaging transducer 220, includes a piezoelectric material, and generates the imaging ultrasound with a power supplied from an external power source. The second matching layer 222 is arranged in front of the second piezoelectric member 221 in the imaging transducer 220, and prevents the ultrasound from being reflected at the surface of the subject 200 by matching an acoustic impedance of the second piezoelectric member 221 with that of the subject 200. The fluid medium 230 is arranged ahead of both the HIFU transducer 210 and the imaging transducer 220, and helps the ultrasounds emitted from the first piezoelectric member 2111 and the second piezoelectric member 221 to smoothly arrive at the subject 200 without losing much of their intensity. A fluid used for the fluid medium 230 includes, for example, water.

The membrane 240 forms a space for accommodating the fluid, and prevents a leakage of the fluid medium 230.

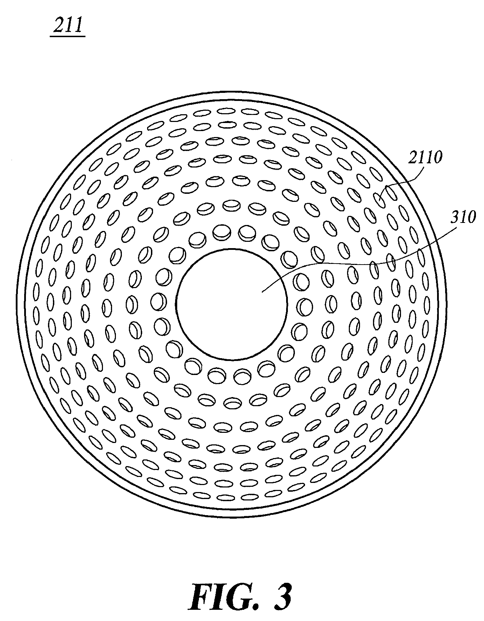

FIG. 3 is a front view of a HIFU emitting unit of a transducer according to some embodiments of the present disclosure.

The HIFU emitting unit 211 has a parabolic shape in which the front of the entire HIFU emitting unit 211 defines the inner circumferential surface. A plurality of the recesses 2110 are evenly arranged in the entire HIFU emitting unit 211. In some embodiments, a scheme of arranging the recesses 2110 includes defining a plurality of virtual circumferences at regular intervals on the HIFU emitting unit 211 and arranging a predetermined number of the recesses 2110 along the circumference. In this case, the total number of the recesses 2110 is appropriately determined considering the number of first piezoelectric members 2111 used in the HIFU transducer 210, the number of the recesses 2110 free of the first piezoelectric member 2111, and the like. The HIFU emitting unit 211 includes a hollow portion 310 at the center, in which the imaging transducer 220 is arranged.

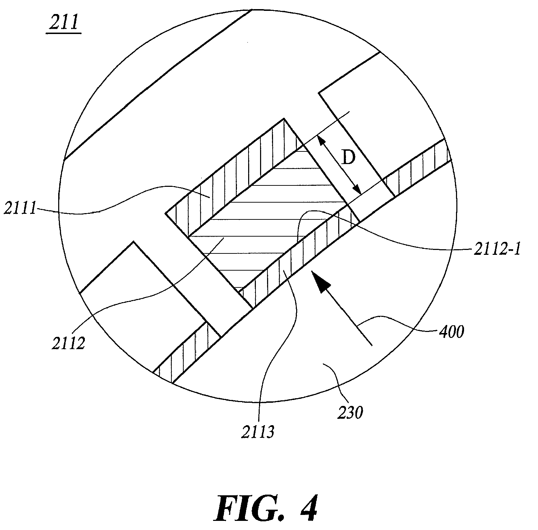

FIG. 4 is a partial cross-sectional view of a HIFU transducer of a structure according to some embodiments of the present disclosure.

When a back-reflected ultrasound 400 reflected at the membrane 240 and the like is rereflected at a first matching layer surface 2112-1 and eventually incident on the imaging transducer 220, the quality of the image acquired by the imaging transducer 220 is degraded. Therefore, if it is designed such that the back-reflected ultrasound 400 is not rereflected at the first matching layer surface 2112-1 but absorbed by the first matching layer 2112, the quality of the image can be improved, which can be achieved by adjusting the thickness of the first matching layer 2112.

In general, the thickness of the first matching layer 2112 with which the back-reflected ultrasound 400 is not reflected at the first matching layer 2112 but absorbed thereby is an odd-number multiple of a quarter of the wavelength of the back-reflected ultrasound 400, to satisfy Equation 7.

$$D=\lambda(2k-1)/4 \qquad \text{Equation 7}$$

where D is the thickness of the first matching layer 2112, $\lambda$ the wavelength of the ultrasound at the first matching layer 2112, and k a positive integer. In this case, the speed of the ultrasound is equal to the speed of the sound wave, and the wavelength of the ultrasound at the first matching layer 2112 is represented by

$$\lambda=\frac{c}{f} \qquad \text{Equation 8}$$

where c is speed of the sound wave in the first matching layer 2112 and f is the frequency of the sound waves.

On the other hand, the back-reflected ultrasound 400 includes both the imaging ultrasound and the treatment ultrasound, and therefore, in order for the back-reflected ultrasound 400 not to be reflected at the first matching layer surface 2112-1 but absorbed by the first matching layer 2112, it is appropriate to equalize thickness DI of the first matching layer 2112 that is set considering the frequency of the imaging ultrasound with thickness DH of the first matching layer 2112 that is set considering the frequency of the treatment ultrasound.

Therefore, the first matching layer 2112 has thickness DI, DH which is determined to be the same as a product from multiplying a quarter of the wavelength of the ultrasound emitted from the imaging transducer 220 by an odd number if the product is equal to a quarter of the wavelength of the ultrasound emitted from the HIFU transducer 210 multiplied by an odd number. Specifically, the thickness (DI, DH) of the first matching layer 2112 should satisfy Equations 1 to 3.

$$\frac{c}{fIc}\times\frac{2n-1}{4}\times 1000=\frac{c}{fHc}\times\frac{2m-1}{4}\times 1000 \qquad \text{Equation 1}$$

$$\frac{c}{fIc} \times \frac{2n-1}{4} \times 1000 = DI$$

Equation 2

$$\frac{c}{fHc} \times \frac{2m-1}{4} \times 1000 = DH$$

Equation 3

where DI is thickness (in millimeters) of the first matching layer 2112 that is set considering the frequency of the imaging ultrasound, DH is the thickness (in millimeters) of the first matching layer 2112 that is set considering the frequency of the treatment ultrasound, c is the speed of sound wave (in m/s) in the first matching layer 2112, fIc is the center frequency (in megahertz) of the ultrasound emitted from the imaging transducer 220, fHc is the center frequency (in megahertz) of the ultrasound emitted from the HIFU transducer 210, and n and m are positive integers.

In the above Equations, the frequency of the ultrasound varies with a predetermined bandwidth, and therefore the thickness of the first matching layer 2112 is determined by using the center frequencies.

For example, when fIc is 3 megahertz and fHc is 1 megahertz, n is 2 when m is 1, and the thickness (DI, DH) of the first matching layer 2112 is (c/4) x 1000 millimeters.

On the other hand, a comparison is made of a first product from multiplying a quarter of the wavelength of the ultrasound emitted from the imaging transducer 220 by an odd number with a second product from multiplying a quarter of the wavelength of the ultrasound emitted from the HIFU transducer 210 by a positive real number excluding an odd number, and if the two products are the same, then the thickness of the first matching layer 2112 is determined to be a third product from multiplying the quarter of the wavelength of the ultrasound emitted from the HIFU transducer 210 by an odd number nearest to the positive real number. Specifically, when n is a positive integer and m is a positive real number excluding integers, the thickness (DI, DH) of the first matching layer 2112 should satisfy Equations 4 to 6.

$$m = 0.5\left(\frac{fHc}{fIc} \times (2n-1) + 1\right)$$

Equation 4

$$-0.25 \leq \frac{m'-m}{m'} \leq 0.25$$

Equation 5

$$\frac{c}{fHc} \times \frac{2m-1}{4} \times 1000 = DI = DH$$

Equation 6

where DI is thickness (in millimeters) of the first matching layer 2112 that is set considering the frequency of the imaging ultrasound, DH is the thickness (in millimeters) of the first matching layer 2112 that is set considering the frequency of the treatment ultrasound, c is the speed of sound wave (in m/s) in the first matching layer 2112, fIc is the center frequency (in megahertz) of the ultrasound emitted from the imaging transducer 220, fHc is the center frequency (in megahertz) of the ultrasound emitted from the HIFU transducer 210, n is a positive integer, m is a positive real number excluding integers, and m' is a positive integer obtained by rounding off m to the nearest integer.

For example, when fIc = 3.75 megahertz and fHc = 1 megahertz,
m = 0.63 when n = 1, and (m' - m) / m' = 0.37,
m = 0.9 when n = 2, and (m' - m) / m' = 0.1, and
m = 1.17 when n = 3, and (m' - m) /m' = 0.17.

When n = 1, Equation 5 is not satisfied, which fails to determine the thickness of the first matching layer 2112. However, when n = 2 or n = 3, Equation 5 is satisfied, which proceeds to determine the thickness of the first matching layer 2112.

In this case, for when c = 1540 m/s, thickness DH of the first matching layer 2112 is
0.3 millimeters when m = 0.9, and

0.5 millimeters when m = 1.17.

With the determined DH satisfying all the above Equations, one can fabricate the first matching layer 2112 for use.

The thickness of the first matching layer 2112 that is determined in the above manner is subject to a reduced performance of the first matching layer 2112 to absorb the back-reflected ultrasound 400 when n is a positive integer and m is a real number excluding integers as compared to a case where both n and m are positive integers. However, both n and m are positive integers limitedly when determining the thickness of the first matching layer 2112, and therefore applying the positive real number m excluding integers to determining the thickness of the first matching layer 2112 ensures an appropriate thickness selection thereof for various flc and fHc values. As represented by the above Equations, the thickness of the first matching layer 2112 is determined by using limited m, so as to ensure that the thickness of the first matching layer 2112 be approximate to an odd-number multiple of the quarter of the wavelength of the back-reflected ultrasound 400.

Although exemplary embodiments of the present disclosure have been described for illustrative purposes, those skilled in the art will appreciate that various modifications, additions and substitutions are possible, without departing from the idea and scope of the claimed disclosure. Accordingly, one of ordinary skill would understand the scope of the claimed disclosure is not to be limited by the explicitly described above embodiments but by the claims and equivalents thereof.

[Industrial Applicability]

As described above, the present disclosure is highly useful for application in various industrial fields. The transducer according to some embodiments of the present disclosure improves the quality of the ultrasound image by extinguishing the ultrasound back-reflected to the HIFU emitting unit or adjusting the thickness of the matching layer.

CROSS-REFERENCE TO RELATED APPLICATION

This application claims priority under 35 U.S.C §119(a) of Patent Application No. 10-2013-0062672, filed on May 31, 2013 in Korea, the entire content of which is incorporated herein by reference. In addition, this non-provisional application claims priority in countries, other than the U.S., with the same reason based on the Korean patent application, the entire content of which is hereby incorporated by reference.

**Claims**

1. A transducer, comprising:

   a high intensity focused ultrasound (HIFU) transducer including a HIFU emitting unit configured to emit a treatment ultrasound; and
   an imaging transducer arranged in proximity of the HIFU transducer and configured to emit an imaging ultrasound, wherein
   the HIFU emitting unit includes at least one recess configured to extinguish whole or a part of the treatment ultrasound and the imaging ultrasound back-reflected to the HIFU emitting unit.

2. The transducer according to claim 1, wherein the recess includes a depressed portion of a circular or polygonal shape with an open axial side.

3. The transducer according to claim 1, wherein
   each of all or some of the at least one recess includes a first piezoelectric member configured to generate the treatment ultrasound, and
   the imaging transducer includes a second piezoelectric member configured to generate the imaging ultrasound.

4. The transducer according to claim 3, wherein
   the at least one recess includes

   a filling member on an outer area of the recess,
   the first piezoelectric member on an inner side of the recess, and
   a first matching layer, between the first piezoelectric member and the filling member, for matching an acoustic impedance of the first piezoelectric member with an acoustic impedance of a subject at which the ultrasound arrives, and

the imaging transducer includes a second matching layer for matching an acoustic impedance of the second piezoelectric member with the acoustic impedance of the subject.

5. The transducer according to claim 4, wherein when a product from multiplying a quarter of a wavelength of the ultrasound emitted from the imaging transducer by an odd number is equal to a wavelength of the ultrasound emitted from the HIFU transducer multiplied by an odd number, the product is determined as a thickness of the first matching layer.

6. The transducer according to claim 4, wherein when a first product from multiplying a quarter of a wavelength of the ultrasound emitted from the imaging transducer by an odd number is equal to a second product from multiplying a quarter of a wavelength of the ultrasound emitted from the HIFU transducer by a positive real number excluding an odd number, a third product from multiplying the quarter of the wavelength of the ultrasound emitted from the HIFU transducer by an odd number nearest to the positive real number is determined as a thickness of the first matching layer.

7. The transducer according to claim 4, wherein the filling member includes rubber or polymer material.

**FIG. 1**

**FIG. 2**

**FIG. 3**

**FIG. 4**

## INTERNATIONAL SEARCH REPORT

| International application No. |
| --- |
| **PCT/KR2013/004835** |

**A.    CLASSIFICATION OF SUBJECT MATTER**

*A61N 7/02(2006.01)i, A61B 8/00(2006.01)i*

According to International Patent Classification (IPC) or to both national classification and IPC

**B.    FIELDS SEARCHED**

Minimum documentation searched (classification system followed by classification symbols)
A61N 7/02; A61N 7/00; A61B 17/22; A61B 17/36; A61H 23/00; A61B 8/00

Documentation searched other than minimum documentation to the extent that such documents are included in the fields searched
Korean Utility models and applications for Utility models: IPC as above
Japanese Utility models and applications for Utility models: IPC as above

Electronic data base consulted during the international search (name of data base and, where practicable, search terms used)
eKOMPASS (KIPO internal) & Keywords: "HIFU", HIFU, high intensity focused ultrasound, image, imaging, image, picture, ultrasonic waves, ultrasonic, transducer, transducer, converter, piezoelectric, dent, penetration, hole

**C.    DOCUMENTS CONSIDERED TO BE RELEVANT**

| Category* | Citation of document, with indication, where appropriate, of the relevant passages | Relevant to claim No. |
| --- | --- | --- |
| X | KR 10-2011-0074326 A (RDS KOREA CO., LTD.) 30 June 2011<br>See abstract; claims 1-4; paragraphs [0001], [0012]-[0020], [0024]-[0026]; and figures 2, 3, 5. | 1-3 |
| A | | 4-7 |
| A | KR 10-2012-0126682 A (ALPINION MEDICAL SYSTEMS CO., LTD.)<br>21 November 2012<br>See abstract; claims 1-4; paragraphs [0001], [0028]-[0034], [0037]-[0041], [0051]-[0056]; and figures 3-4. | 1-7 |
| A | KR 10-2013-0055972 A (ALPINION MEDICAL SYSTEMS CO., LTD.) 29 May 2013<br>See abstract; claims 1-3; paragraphs [0001], [0022]-[0025], [0029]-[0030], [0033]-[0035], [0070]; and figure 8. | 1-7 |
| A | US 2005-0015024 A1 (BABAEV, E.) 20 January 2005<br>See abstract; claims 1-3; paragraphs [0001], [0013]-[0014], [0026]-[0028]; and figures 1-2C. | 1-7 |
| A | JP 06-189973 A (TOSHIBA KABUSHIKI KAISHA) 12 July 1994<br>See abstract; claim 1; paragraphs [0008], [0011], [0020], [0024]; and figures 1, 12. | 1-7 |

☐  Further documents are listed in the continuation of Box C.          ☒  See patent family annex.

| | |
| --- | --- |
| *    Special categories of cited documents:<br>"A"  document defining the general state of the art which is not considered to be of particular relevance<br>"E"  earlier application or patent but published on or after the international filing date<br>"L"  document which may throw doubts on priority claim(s) or which is cited to establish the publication date of another citation or other special reason (as specified)<br>"O"  document referring to an oral disclosure, use, exhibition or other means<br>"P"  document published prior to the international filing date but later than the priority date claimed | "T"  later document published after the international filing date or priority date and not in conflict with the application but cited to understand the principle or theory underlying the invention<br>"X"  document of particular relevance; the claimed invention cannot be considered novel or cannot be considered to involve an inventive step when the document is taken alone<br>"Y"  document of particular relevance; the claimed invention cannot be considered to involve an inventive step when the document is combined with one or more other such documents, such combination being obvious to a person skilled in the art<br>"&"  document member of the same patent family |

| Date of the actual completion of the international search | Date of mailing of the international search report |
| --- | --- |
| 25 FEBRUARY 2014 (25.02.2014) | **26 FEBRUARY 2014 (26.02.2014)** |

| Name and mailing address of the ISA/KR<br>Korean Intellectual Property Office<br>Government Complex-Daejeon, 189 Seonsa-ro, Daejeon 302-701,<br>Republic of Korea<br>Facsimile No.  82-42-472-7140 | Authorized officer<br><br>Telephone No. |
| --- | --- |

Form PCT/ISA/210 (second sheet) (July 2009)

INTERNATIONAL SEARCH REPORT
Information on patent family members

International application No.

**PCT/KR2013/004835**

| Patent document cited in search report | Publication date | Patent family member | Publication date |
|---|---|---|---|
| KR 10-2011-0074326 A | 30/06/2011 | NONE | |
| KR 10-2012-0126682 A | 21/11/2012 | KR 10-1259381 B1 | 30/04/2013 |
| | | WO 2012-153888 A1 | 15/11/2012 |
| KR 10-2013-0055972 A | 29/05/2013 | WO 2013-077506 A1 | 30/05/2013 |
| US 2005-0015024 A1 | 20/01/2005 | US 2003-0171701 A1 | 11/09/2003 |
| JP 06-189973 A | 12/07/1994 | NONE | |

Form PCT/ISA/210 (patent family annex) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020130062672